# EUROPEAN PATENT APPLICATION

(11) **EP 4 620 489 A1**
(43) Date of publication of application: **24.09.2025**
(21) Application number: 24841410.4
(22) Date of filing: 29.08.2024
(51) Int. Cl.: A61L 9/03, A61L 9/14

(54) **LIQUID STORAGE ASSEMBLY AND AROMA DIFFUSER**

(30) Priority: 01.02.2024 CN 202410145431
(71) Applicant: Shenzhen Fenyue Technology Co., Ltd, Shenzhen, Guangdong 518101 (CN)
(72) Inventor: WEI, Chunhua, Shenzhen, Guangdong 518101 (CN); WANG, Lingquan, Shenzhen, Guangdong 518101 (CN); AN, Pengzhan, Shenzhen, Guangdong 518101 (CN); LI, Weiguo, Shenzhen, Guangdong 518101 (CN); JIANG, Wennan, Shenzhen, Guangdong 518101 (CN); SUN, Congyang, Shenzhen, Guangdong 518101 (CN)
(74) Representative: Steffens, Adrian
(86) International application number: PCT/CN2024/115602
(87) International publication number: WO 2025/161374

(57) **Abstract**

Disclosed are a liquid storage component and an aromatherapy device, belonging to the technical field of aromatherapy devices. **The** liquid storage component is provided with a liquid storage chamber; the liquid storage component includes a lower shell, which encloses at least a portion of the liquid storage chamber and includes a liquid guide portion and a support portion; a microporous structure is provided inside the liquid guide portion and is in communication with the liquid storage chamber, and the support portion is of a dense structure and is integrally formed with the liquid guide portion; essential oil enters the microporous structure from the liquid storage chamber and is delivered to a volatilization surface of the liquid guide portion by the microporous structure, and the essential oil can volatilize and diffuse on the volatilization surface into an external environment. **The** support portion can provide support for the liquid guide portion. **The** one-piece structure of the lower shell does not need mounting components, which can prevent failure of the liquid storage component due to poor corrosion resistance of the mounting components in corrosive essential oils, making the liquid storage component applicable to various scenarios.

## Description

### CROSS REFERENCE TO RELATED APPLICATION

The present application claims priority to Chinese Patent Application No. 2024101454310 filed on February 1, 2024, which is incorporated herein by reference in its entirety.

### TECHNICAL FIELD

The present application relates to the technical field of aromatherapy devices, in particular to a liquid storage component and an aromatherapy device.

### BACKGROUND

A liquid guide made of cotton, wood, or the like is usually used in an existing aromatherapy device to deliver an essential oil stored in an oil storage bin, and the liquid guide is mounted on the oil storage bin through a plastic bracket. Some corrosive essential oils can corrode and deform the plastic bracket and other components, resulting in failure of the oil storage bin. Therefore, the oil storage bin in these solutions can only be applied to non-corrosive essential oils, and its application scenario is limited.

### SUMMARY

The present application provides a liquid storage component and an aromatherapy device, which can solve the technical problem that the application scenario of the liquid storage component is limited.

To solve the above technical problem, on the one hand, the present application provides a liquid storage component used for an aromatherapy device. The liquid storage component is provided with a liquid storage chamber for storing an essential oil; and the liquid storage component includes a lower shell, the lower shell encloses at least a portion of the liquid storage chamber, the lower shell includes a liquid guide portion and a support portion, a microporous structure is provided inside the liquid guide portion and is in communication with the liquid storage chamber, the support portion is of a dense structure and is integrally formed with the liquid guide portion, the essential oil enters the microporous structure from the liquid storage chamber and is delivered to a volatilization surface of the liquid guide portion by the microporous structure, and the essential oil can volatilize and diffuse on the volatilization surface into an external environment.

On the other hand, the present application provides an aromatherapy device, including the liquid storage component as described above and a heating portion, the heating portion is arranged on the liquid guide portion, and the heating portion is used for heating the essential oil, so that the essential oil is heated at the liquid guide portion to volatilize and diffuse into an external environment.

According to the liquid storage component provided in the present application, the liquid storage component includes a lower shell, the lower shell encloses at least a portion of the liquid storage chamber, the lower shell includes a liquid guide portion and a support portion, a microporous structure is provided inside the liquid guide portion and is in communication with the liquid storage chamber, and the support portion is of a dense structure and is integrally formed with the liquid guide portion, so that the support portion can provide support for the liquid guide portion; and the lower shell forms a one-piece structure, which does not need mounting components and can prevent failure of the liquid storage component due to poor corrosion resistance of the mounting components in corrosive essential oils, making the liquid storage component applicable to various scenarios.

### BRIEF DESCRIPTION OF THE DRAWINGS

To describe the technical solutions in the embodiments of the present application more clearly, the following briefly introduces the accompanying drawings required for use in the description of the embodiments. Apparently, the accompanying drawings in the following description show only some embodiments of the present application, and those of ordinary skill in the art can further derive other drawings from the accompanying drawings without any creative effort.
- FIG. 1: is a cross-sectional structure diagram of an embodiment of a liquid storage component provided in the present application from a perspective;
- FIG. 2: is a cross-sectional structure diagram of an embodiment of the liquid storage component provided in the present application from another perspective;
- FIG. 3: is a cross-sectional structure diagram of an embodiment of a lower shell provided in the present application;
- FIG. 4: is a structure diagram of an embodiment of a support portion provided in the present application;
- FIG. 5: is a structure diagram of an embodiment of a liquid guide portion provided in the present application;
- FIG. 6: is a cross-sectional structure diagram of an embodiment of an aromatherapy device provided in the present application from a perspective;
- FIG. 7: is a cross-sectional structure diagram of an embodiment of the aromatherapy device provided in the present application from another perspective;
- FIG. 8: is a partial cross-sectional structure diagram of an embodiment of the aromatherapy device provided in the present application from a perspective;
- FIG. 9: is a structure diagram of an embodiment of the lower shell provided in the present application;
- FIG. 10: is an exploded structure diagram of an embodiment of the aromatherapy device provided in the present application;
- FIG. 11: is a cross-sectional structure diagram of an embodiment of the aromatherapy device provided in the present application; and
- FIG. 12: is a partial cross-sectional structure diagram of an embodiment of the aromatherapy device provided in the present application.

### DETAILED DESCRIPTION

The present application will be further described in detail below in conjunction with the accompanying drawings and embodiments. It should be particularly noted that the following embodiments are only used for illustrating the present application, but do not limit the scope of the present application. Similarly, the following embodiments are only some embodiments of the present application, not all embodiments. All other embodiments obtained by those of ordinary skill in the art without creative effort fall within the scope of protection of the present application.

In the description of the present application, "a plurality of" means at least two, such as two or three, unless otherwise specified. The terms "first", "second", and "third" used in the embodiments of the present application are for descriptive purposes only and should not be understood as indicating or implying relative importance or implying a quantity of indicated technical features. Therefore, the features limited to "first", "second", and "third" may explicitly or implicitly include at least one of these features. All directional indications (such as up, down, left, right, front, and back) in the embodiments of the present application are only used for explaining relative positional relationships, movement situations, and the like of components at a specific posture (as shown in the accompanying drawings), and if the specific posture changes, the directionality indication changes accordingly. The terms "include" and "have" and any variations thereof in the embodiments of the present application are intended to cover non-exclusive inclusions. For example, a process, method, system, product, or device that includes a series of steps or units is not limited to the listed steps or units, but optionally further includes unlisted steps or units, or optionally further includes other inherent steps or units of the process, method, product, or device.

The phrase "embodiment" referred to herein means that specific features, structures, or characteristics described in conjunction with the embodiment may be included in at least one embodiment of the present application. The phrase at various locations in the specification does not necessarily refer to the same embodiment, or an independent or alternative embodiment exclusive of another embodiment. Those skilled in the art understand, in explicit and implicit manners, that an embodiment described herein may be combined with another embodiment.

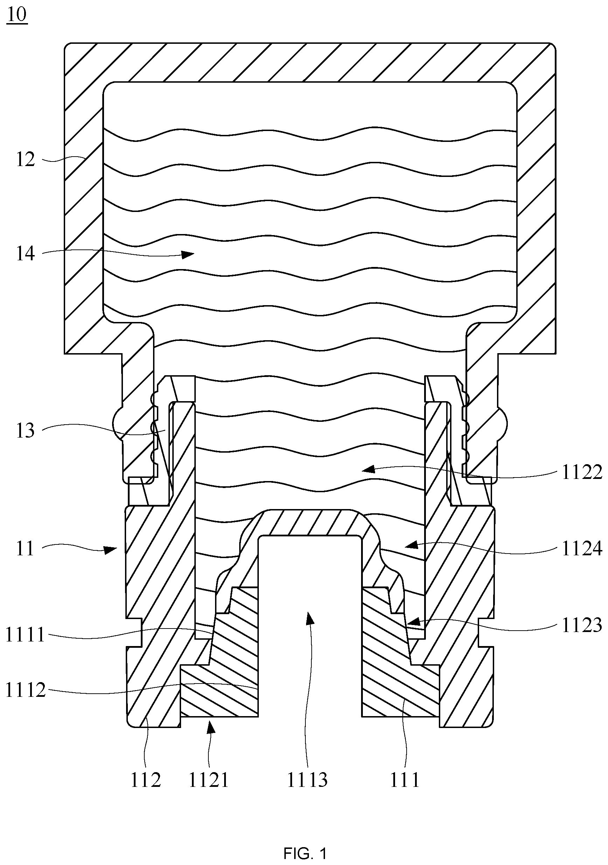

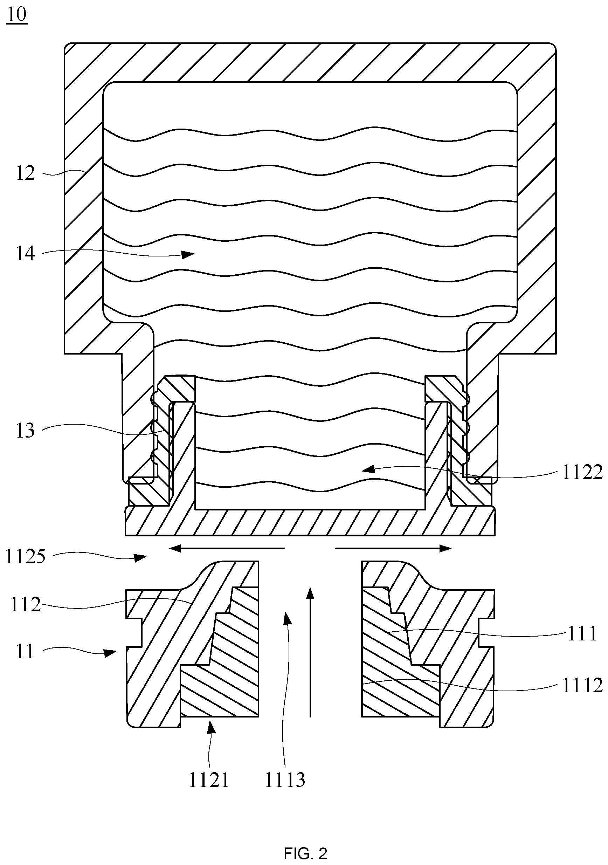

The present application provides a liquid storage component. With reference to FIG. 1 and FIG. 2, the liquid storage component 10 is provided with a liquid storage chamber 14 for storing an essential oil. Optionally, in the assembling process of the liquid storage component 10, the essential oil may be first injected into the liquid storage chamber 14, and then the liquid storage chamber 14 is closed to seal the essential oil inside the liquid storage component 10; or an oil injection hole for injecting the essential oil is formed on the liquid storage chamber 14, the essential oil can be injected into the liquid storage chamber 14 after assembling and before the product leaves the factory, or during use as required, and the oil injection hole is sealed to seal the essential oil inside the liquid storage component 10. Under normal circumstances, the essential oil is sealed in the liquid storage chamber 14 and is not in direct contact with air, which can prevent the essential oil from being oxidized and deteriorated.

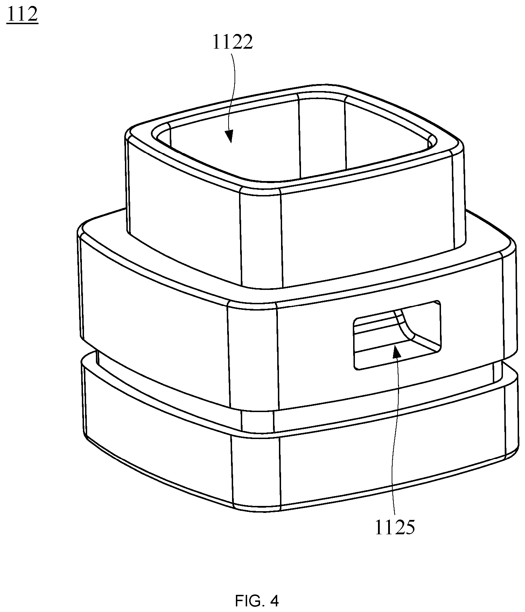

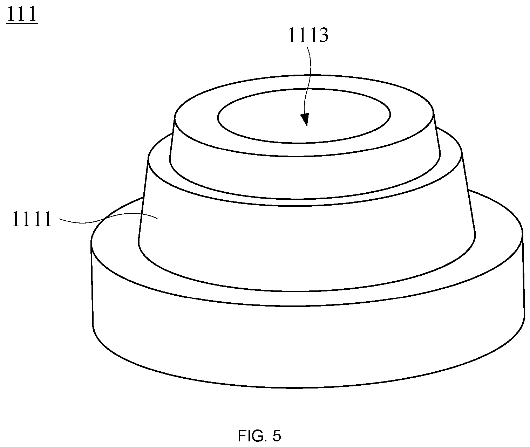

With reference to FIGs. 1-5, the liquid storage component 10 includes a lower shell 11, and the lower shell 11 encloses at least a portion of the liquid storage chamber 14. The lower shell 11 includes a liquid guide portion 111 and a support portion 112, a microporous structure is provided inside the liquid guide portion 111 and is in communication with the liquid storage chamber 14, and the support portion 112 is of a dense structure and is integrally formed with the liquid guide portion 111. The liquid guide portion 111 is used for delivering the essential oil. The dense structure of the support portion 112 indicates that the support portion 112 has a smaller porosity than the liquid guide portion 111, so that the strength of the support portion 112 is higher. Because the liquid guide portion 111 is provided with the microporous structure required for delivering the essential oil, the strength of the liquid guide portion 111 is reduced, and there is a risk of damage to the liquid guide portion 111 in the assembling process of the liquid storage component 10. By configuring the support portion 112 to be of a dense structure and be integrally formed with the liquid guide portion 111, on the one hand, the support portion 112 can provide support for the liquid guide portion 111, thereby reducing the risk of damage to the liquid guide portion 111 in the assembling process of the liquid storage component 10; and on the other hand, the support portion 112 is integrally formed with the liquid guide portion 111, so that the lower shell 11 forms a one-piece structure, which does not need mounting components and can prevent failure of the liquid storage component 10 due to poor corrosion resistance of the mounting components in corrosive essential oils, making the liquid storage component 10 applicable to various scenarios.

The essential oil enters the liquid guide portion 111 from the microporous structure, and the essential oil can volatilize and diffuse at the liquid guide portion 111 into an external environment. Optionally, the liquid guide portion 111 has a liquid guide surface 1111 and a volatilization surface 1112, where the volatilization surface 1112 may be a portion of the outer surface of the liquid guide portion 111 or the surface of the microporous structure. The essential oil enters the microporous structure from the liquid storage chamber 14 and is delivered to the volatilization surface 1112 of the liquid guide portion 111 by the microporous structure, and the essential oil can volatilize and diffuse on the volatilization surface 1112. The essential oil may directly enter the microporous structure from the liquid storage chamber 14, for example, the liquid guide surface 1111 is in communication with the liquid storage chamber 14, and the essential oil is delivered from the liquid guide surface 1111 to the volatilization surface 1112. Alternatively, the essential oil may indirectly enter the microporous structure from the liquid storage chamber 14, for example, other components are further provided inside the liquid storage chamber 14, and the essential oil indirectly enters the microporous structure through the other components. Optionally, the essential oil volatilizes and diffuses from the volatilization surface 1112 when heated, that is, the essential oil hardly volatilizes or volatilizes very slowly at room temperature. Therefore, the volatilization of the essential oil can be controlled or stopped by heating, and the volatilization rate of the essential oil can also be controlled by adjusting the heating temperature, to satisfy optimal fragrance emitting conditions for different types of essential oils.

According to the liquid storage component 10 provided in the present application, the liquid storage component 10 includes a lower shell 11, the lower shell 11 encloses at least a portion of the liquid storage chamber 14, the lower shell 11 includes a liquid guide portion 111 and a support portion 112, a microporous structure is provided inside the liquid guide portion 111 and is in communication with the liquid storage chamber 14, and the support portion 112 is of a dense structure and is integrally formed with the liquid guide portion 111, so that the support portion 112 can provide support for the liquid guide portion 111; and the lower shell 11 forms a one-piece structure, which does not need mounting components and can prevent failure of the liquid storage component 10 due to poor corrosion resistance of the mounting components in corrosive essential oils, making the liquid storage component 10 applicable to various scenarios.

Optionally, the material of the liquid guide portion 111 and the support portion 112 may be ceramic or glass, and these materials have good corrosion resistance and can be used for corrosive essential oils, making the liquid storage component 10 applicable to various scenarios. In one embodiment, both the liquid guide portion 111 and the support portion 112 are made of ceramic, the liquid guide portion 111 is connected to the support portion 112, and the liquid guide portion 111 and the support portion 112 are co-fired into a one-piece structure at a preset temperature. Optionally, the preparation material for the liquid guide portion 111 and the support portion 112 is one of silicon oxide, aluminum oxide, zirconium oxide, or silicon carbide. The liquid guide portion 111 and the support portion 112 form a one-piece structure by co-firing, where the co-firing enables the liquid guide portion 111 and the support portion 112 to form a molecular bond on a bonding surface, thereby increasing the connection strength between the two.

Specifically, in the process of fabricating the lower shell 11, a mold is first formed according to the preset shape of the support portion 112, ceramic slurry is injected into the mold to form a support portion blank, the support portion blank is sintered at a high temperature in a high-temperature furnace, and the sintered support portion blank is machined, polished, or the like to achieve the support portion 112 with designed dimensional accuracy. Then, a mold is formed according to the preset shape of the liquid guide portion 111, the pre-sintered support portion 112 is placed in the mold, ceramic slurry is injected into the mold to form a lower shell blank including the support portion 112, and the lower shell blank is sintered to obtain the lower shell 11 with a one-piece structure prepared from the ceramic material. A pore-forming agent is added to the ceramic slurry, so that the sintered liquid guide portion 111 has pores inside, and its porosity and pore size meet design requirements. In the process of fabricating the lower shell 11, the support portion 112 is pre-sintered. On the one hand, the sintering temperature of the support portion 112 is not limited by the sintering temperature of the liquid guide portion 111, so that the sintering temperature of the support portion 112 is higher to increase the strength of the support portion 112. On the other hand, the pre-sintered support portion 112 can be used as a portion of the mold for the liquid guide portion 111 without dismantling, which reduces the quantity of molds used in fabricating the liquid guide portion 111 and also reduces the dismantling workload of the mold after the lower shell 11 is sintered.

Optionally, the sintering temperature of the support portion 112 is greater than or equal to 1600°C, and the sintering temperature of the liquid guide portion 111 is 500-800°C. The sintering temperature of the support portion 112 is significantly higher than that of the liquid guide portion 111, which can increase the strength of the support portion 112, so that the support portion 112 can provide good support for the liquid guide portion 111.

Optionally, the liquid guide portion 111 has a pore size of 10-30 µm. If the pore size of the liquid guide portion 111 is less than 10 µm, the small pore size of the liquid guide portion 111 will limit the supply rate of the essential oil, making it difficult to meet the volatilization rate requirements of the volatilization surface 1112. If the pore size of the liquid guide portion 111 is greater than 30 µm, the large pore size of the liquid guide portion 111 will reduce the oil locking capability of the lower shell 11, resulting in leakage of the essential oil. For example, the pore size of the liquid guide portion 111 is 10 µm, 15 µm, 20 µm, 25 µm, 30 µm, etc., which is not specifically limited here.

Optionally, the liquid guide portion 111 has a porosity of 30-50%. If the porosity of the liquid guide portion 111 is less than 30%, it will also limit the supply rate of the essential oil, making it difficult to meet the volatilization rate requirements of the volatilization surface 1112. If the porosity of the liquid guide portion 111 is greater than 50%, the large porosity of the liquid guide portion 111 will reduce the local strength of the lower shell 11, and the position of the liquid guide portion 111 is prone to phenomena such as powder falling. Specifically, the porosity of the liquid guide portion 111 may be 30%, 35%, 40%, 45%, 50%, etc.

With reference to FIGs. 1-3, in one embodiment, the support portion 112 is provided with a mounting chamber 1121, the liquid guide portion 111 is nested in the mounting chamber 1121, and an outer wall of the liquid guide portion 111 is attached to an inner wall of the mounting chamber 1121. In this way, the liquid guide portion 111 and the support portion 112 form an inner and outer nested structure. The high-strength support portion 112 is nested outside the liquid guide portion 111 to provide good support for the liquid guide portion 111, so as to prevent damage to the liquid guide portion 111 in the assembling process.

In one embodiment, as shown in FIG. 1, the support portion 112 is provided with a liquid guide chamber 1122 and a liquid guide hole 1123, the liquid guide chamber 1122 forms at least a portion of the liquid storage chamber 14, the liquid guide hole 1123 connects the liquid guide chamber 1122 with the liquid guide portion 111, and the essential oil enters the microporous structure via the liquid guide hole 1123. The liquid guide hole 1123 may be arranged near a bottom of the liquid storage chamber 14, and the essential oil in the liquid storage chamber 14 flows through the liquid guide hole 1123 to the liquid guide portion 111, which can reduce the residual amount of the essential oil in the liquid storage chamber 14.

With continued reference to FIG. 1, in one embodiment, the support portion 112 is provided with a liquid guide groove 1124. In at least one conventional posture, the liquid guide groove 1124 is located at a bottom of the liquid guide chamber 1122, and the liquid guide groove 1124 is arranged on a periphery of the liquid guide chamber 1122. The conventional posture refers to a design posture of the liquid storage component 10 in normal use. For example, the liquid storage component 10 is roughly vertically placed, the liquid storage chamber 14 is located at an upper part of the liquid storage component 10, and the essential oil can flow from the liquid storage chamber 14 to the liquid guide portion 111 under the action of gravity. The liquid guide groove 1124 is arranged at the bottom of the liquid guide chamber 1122, and the essential oil can converge in the liquid guide groove 1124 under the action of gravity and then flow to the liquid guide portion 111 through the liquid guide hole 1123 formed on a wall of the liquid guide groove 1124, so that the essential oil at the bottom of the liquid storage chamber 14 can be fully utilized and the residual amount of the essential oil in the liquid storage chamber 14 can be reduced. The liquid guide hole 1123 is formed in a partial area of the wall of the liquid guide groove 1124, and the liquid guide portion 111 seals the liquid guide hole 1123. In this way, on the one hand, the liquid guide hole 1123 forms an essential oil supply channel, and the essential oil in the liquid storage chamber 14 can flow through the liquid guide hole 1123 to the liquid guide portion 111; on the other hand, the liquid guide hole 1123 is formed in the partial area of the wall of the liquid guide groove 1124, and the opening of the liquid guide hole 1123 is smaller than the liquid guide groove 1124, which facilitates adjustment on the rate of essential oil supply and prevents essential oil leakage; in addition, the sealing of the liquid guide portion 111 enhances the sealing property of the liquid storage chamber 14, so that the essential oil is sealed in the liquid storage chamber 14 and is not in direct contact with air, which can prevent the essential oil from being oxidized and deteriorated.

Optionally, in one embodiment, as shown in FIGs. 1-5, the support portion 112 is provided with an air outlet channel 1125, the liquid guide portion 111 is provided with a volatilization space 1113, the volatilization space 1113 is in communication with the air outlet channel 1125, and an inner wall, near the volatilization space 1113, of the liquid guide portion 111 forms the volatilization surface 1112. By providing the volatilization space 1113 in the liquid guide portion 111, a hollow space is formed in the liquid guide portion 111. The volatilization space 1113 can be used for accommodating a heating element. When the heating element is arranged in the volatilization space 1113, the volatilization surface 1112 surrounds the heating element to increase the heating area of the essential oil and uniformly heat the essential oil in all directions, which can improve the volatilization efficiency of the essential oil and reduce energy consumption. The essential oil in the liquid guide portion 111 is heated to volatilize into the volatilization space 1113 and the air outlet channel 1125, thereby diffusing into the external environment. Optionally, the heat from the volatilization space 1113 heats the essential oil in the liquid guide portion 111, so that the essential oil is heated to volatilize. When the heating element heats the essential oil, gas around the heating element in the volatilization space 1113 also expands due to heating, and the gas will flow outward. The volatilization space 1113 is in communication with the air outlet channel 1125, the gas flowing outward flows out of the air outlet channel 1125, and external gas enters the volatilization space 1113 from one end away from the liquid storage chamber 14, to form a connected airflow channel, as shown by straight arrow directions in FIG. 2, so as to prevent the outflowing gas from flowing towards the inflowing gas, and to make the flow of the essential oil after volatilization smoother to facilitate the diffusion of the essential oil.

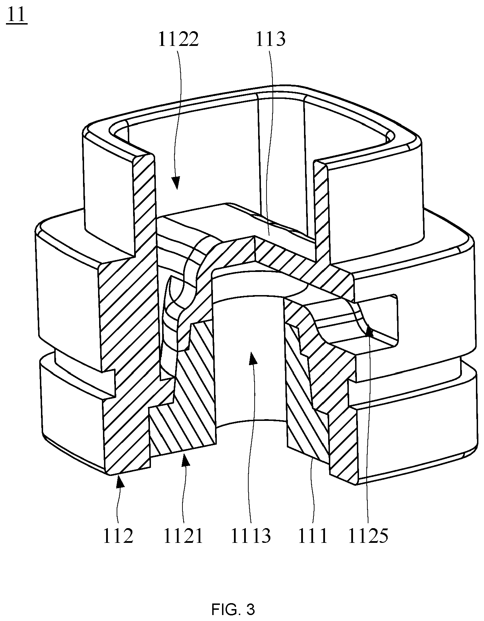

With reference to FIG. 3, in one embodiment, the lower shell 11 further includes a partition 113, the partition 113 is located inside the support portion 112 and connected above the liquid guide portion 111, and the liquid storage chamber 14 and the air outlet channel 1125 are located on two sides of the partition 113 respectively. In at least one conventional posture, at least a portion of the partition 113 forms the bottom of the liquid storage chamber 14, thereby enclosing, together with the support portion 112, an opening through which the essential oil enters the liquid guide portion 111. The support portion 112 may be roughly cylindrical, the partition 113 is arc-shaped, and a sunken arc surface of the partition 113 and an inner wall of the support portion 112 enclose the liquid guide groove 1124 for gathering the essential oil. The air outlet channel 1125 is formed on a side wall of the support portion 112, and the extension direction of the air outlet channel 1125 is the same as that of the arc-shaped groove of the partition 113, which is conducive to guiding the essential oil to volatilize and diffuse through the air outlet channel 1125.

In one embodiment, as shown in FIG. 1 and FIG. 2, the liquid storage component 10 includes an upper shell 12 and a sealing element 13. The lower shell 11 is connected to one end of the upper shell 12, the lower shell 11 and the upper shell 12 enclose the liquid storage chamber 14, and the sealing element 13 is arranged between the lower shell 11 and the upper shell 12 to seal the liquid storage chamber 14. Optionally, the material of the upper shell 12 may be ceramic, glass, or corrosion-resistant silicone, and the sealing element 13 may be made of corrosion-resistant silicone or rubber, making the liquid storage component 10 applicable to various essential oils.

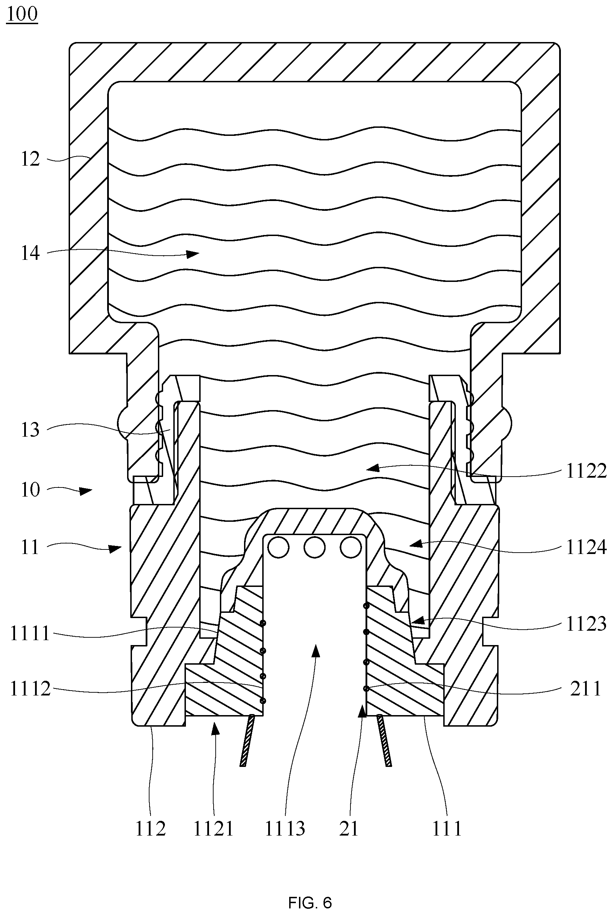

The present application provides an aromatherapy device. With reference to FIGs. 6-9, the aromatherapy device 100 may include the liquid storage component 10 as described above and a heating element 21. The heating element 21 has a heating portion 211, and the heating portion 211 is arranged on the liquid guide portion 111. The heating portion 211 is used for heating an essential oil. For example, under the condition of power-on, the heating portion 211 can generate heat to heat the essential oil, so that the essential oil is heated at the liquid guide portion 111 to volatilize and diffuse into an external environment. The temperature of the essential oil increases after being heated, and the volatilization of the essential oil accelerates. The heating portion 211 is configured to heat the essential oil, so that the essential oil can quickly volatilize and diffuse at the liquid guide portion 111 into the external environment. In addition, the heating portion 211 is arranged on the liquid guide portion 111, and the support portion 112 is integrally formed with the liquid guide portion 111, so that the heating portion 211, the liquid guide portion 111, and the support portion 112 are integrated to form a relatively independent module, which facilitates modular assembly and improves production efficiency.

The heating portion 211 has a resistance value. In one embodiment, the resistance value of the heating portion 211 is 0.2-5 Ω. Research shows that if the resistance value of the heating portion 211 is less than 0.2 Ω, the resistance value of the heating portion 211 is relatively small, and under the condition of a certain voltage applied to the heating portion 211, the heating power of the heating portion 211 is high, and the heating temperature is high, which may damage ingredients in the essential oil and make it difficult to truly restore the aroma of the essential oil; or if the resistance value of the heating portion 211 is greater than 5 Ω, the resistance value of the heating portion 211 is relatively large, and under the condition of a certain voltage applied to the heating portion 211, the heating power of the heating portion 211 is low, the heating temperature is low, and the volatilization rate of the essential oil is small, making it difficult to quickly volatilize and diffuse the essential oil at the liquid guide portion 111 into the external environment. Specifically, the resistance value of the heating portion 211 may be 0.2 Ω, 0.5 Ω, 1.0 Ω, 1.5 Ω, 2.0 Ω, 2.5 Ω, 3.0 Ω, 3.5 Ω, 4.0 Ω, 4.5 Ω, 5.0 Ω, etc., which is not specifically limited here.

Optionally, the heating portion 211 is embedded in the liquid guide portion 111. When the heating portion 211 generates heat, the liquid guide portion 111 in its adjacent area and the essential oil in the liquid guide portion 111 are heated, so that the essential oil is heated at the liquid guide portion 111 to volatilize and diffuse into the external environment. Specifically, in the process of fabricating the lower shell 11, a mold is first formed according to the preset shape of the support portion 112, ceramic slurry is injected into the mold to form a support portion blank, the support portion blank is sintered at a high temperature in a high-temperature furnace, and the sintered support portion blank is machined, polished, or the like to achieve the support portion 112 with designed dimensional accuracy. Then, a mold is formed according to the preset shape of the liquid guide portion 111, the pre-formed heating portion 211 and support portion 112 are placed in the mold, and ceramic slurry is injected into the mold, where the heating portion 211 is embedded in the ceramic slurry to form a lower shell blank including the support portion 112 and the heating portion 211, and the lower shell blank is sintered to obtain the lower shell 11.

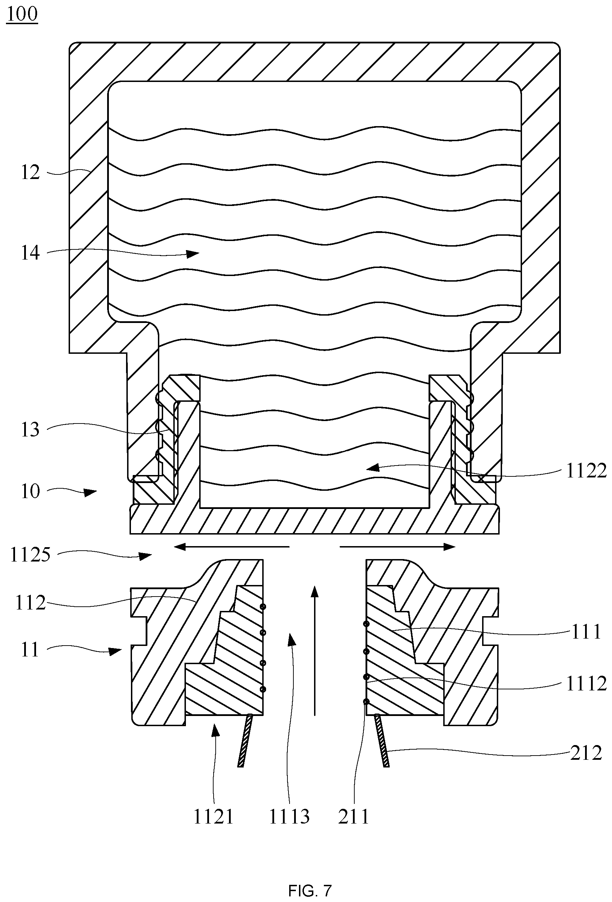

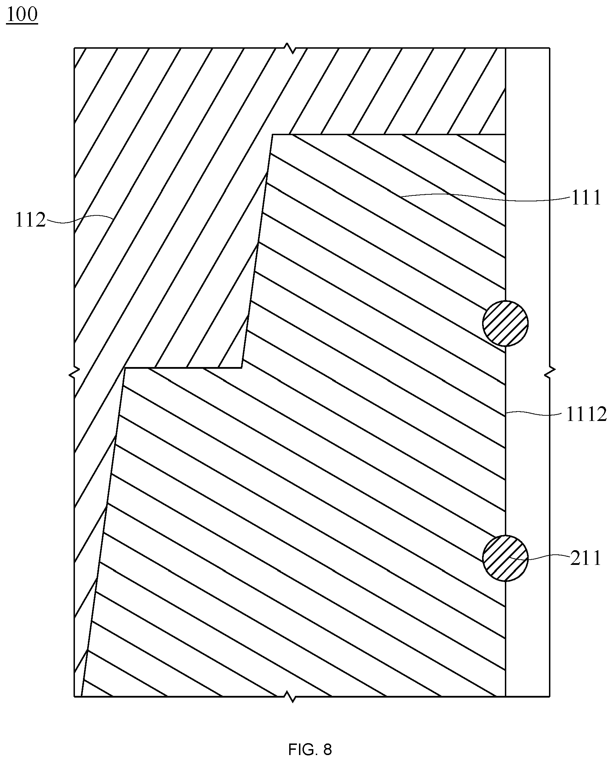

With reference to FIG. 7 and FIG. 8, in one embodiment, the heating portion 211 is embedded on the volatilization surface 1112, a surface of the heating portion 211 is at least partially exposed to the volatilization surface 1112, and the heating portion 211 heats the essential oil in the liquid guide portion 111, so that the essential oil is heated to volatilize into the volatilization space 1113 and the air outlet channel 1125 and diffuse into the external environment. By exposing the surface of the heating portion 211 at least partially to the volatilization surface 1112, the surface of the portion of the heating portion 211 is exposed to the volatilization space 1113. When the heating portion 211 generates heat, the liquid guide portion 111 in its adjacent area and the essential oil in the liquid guide portion 111 are heated. Because the surface of the portion of the heating portion 211 exposed to the volatilization space 1113 is not shielded by the liquid guide portion 111, the essential oil can directly enter the volatilization space 1113 after volatilization, which can improve heating efficiency and facilitate rapid volatilization of the essential oil.

Optionally, the heating portion 211 has a grid structure. For example, the grid heating portion 211 with through holes can be formed from a thin metal sheet by etching or the like. The heating portion 211 is embedded on the volatilization surface 1112, which can increase the contact area between the heating portion 211 and the liquid guide portion 111, thereby improving heating efficiency.

In one embodiment, as shown in FIG. 7, the heating portion 211 has a strip structure. An outer contour of the volatilization space 1113 is columnar, such as cylindrical or prismatic, and the shape of the volatilization space 1113 is relatively regular, which can facilitate the processing of the mold for fabricating the liquid guide portion 111. The heating portion 211 is spirally bent and arranged on the volatilization surface 1112, which can make the distribution of the heating portion 211 on the volatilization surface 1112 more uniform, thereby improving the uniformity of heating.

In one embodiment, the area of a cross-section, perpendicular to an extension direction of the heating portion 211, of the heating portion 211 exposed to the volatilization surface 1112 accounts for 30%-70% of the cross-sectional area. If the area of the cross-section of the heating portion 211 exposed to the volatilization surface 1112 is greater than 70% of the cross-sectional area, the area of the cross-section of the heating portion 211 embedded in the volatilization surface 1112 is relatively small, and the heating portion 211 is prone to detach from the liquid guide portion 111, which will reduce the reliability of the aromatherapy device 100. If the area of the cross-section of the heating portion 211 exposed to the volatilization surface 1112 is less than 30% of the cross-sectional area, the area of the cross-section of the heating portion 211 exposed to the volatilization surface 1112 is relatively small, which will affect the heating efficiency of the heating portion 211 and make it difficult to achieve rapid volatilization of the essential oil. Specifically, the area of the cross-section of the heating portion 211 exposed to the volatilization surface 1112 may account for 30%, 40%, 50%, 60%, or 70% of the cross-sectional area.

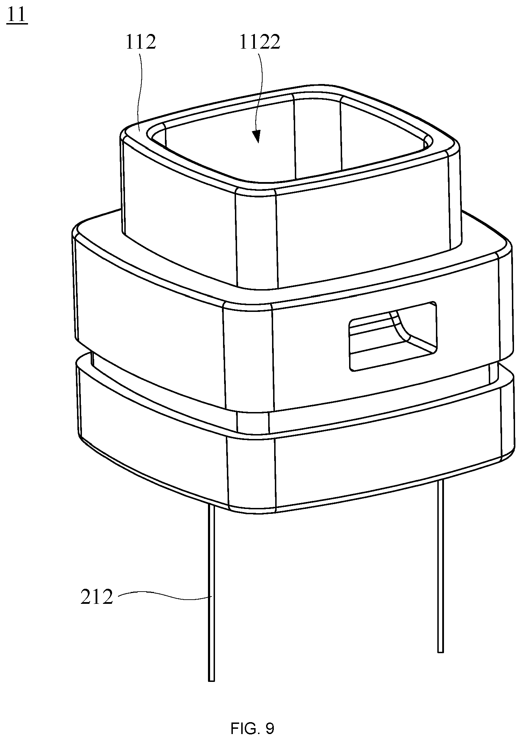

With reference to FIG. 7 and FIG. 9, in one embodiment, the heating element 21 further includes a pin portion 212, and the pin portion 212 is connected with the heating portion 211 into a one-piece structure. The pin portion 212 and the heating portion 211 may have strip structures. The pin portion 212 is used for being electrically connected to an external power source. For example, the pin portion 212 and the heating portion 211 may be simultaneously processed from metal wires. The one-piece structure of the pin portion 212 and the heating portion 211, on the one hand, can improve the connection reliability between the pin portion 212 and the heating portion 211; and on the other hand, the pin portion 212 is further integrated to the lower shell 11, which improves the integration of the lower shell 11 and facilitates modular assembly, thereby improving production efficiency.

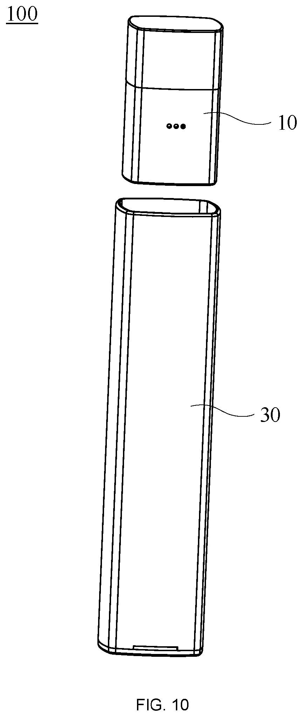

With reference to FIGs. 10-12, the aromatherapy device 100 may include a liquid storage component 10 and a heating component 20, the heating component 20 is provided with a heating element 21, and the heating element 21 can heat an essential oil, so that the essential oil volatilizes and diffuses from the liquid guide portion 111 into an external environment. Optionally, the heating element 21 has a heating portion 211, and the heating portion 211 is arranged on the liquid guide portion 111, so that the heating portion 211, the liquid guide portion 111, and the support portion 112 are integrated to form a relatively independent module, which facilitates modular assembly and improves production efficiency. Alternatively, a heating area of the heating element 21 is spaced apart from the liquid guide portion 111, and the heating element 21 heats the essential oil through non-contact radiation heating, so that the essential oil is heated more uniformly, and there will be no damage to the ingredients in the essential oil due to excessively high local heating temperature. Therefore, the aroma of the essential oil can be truly restored.

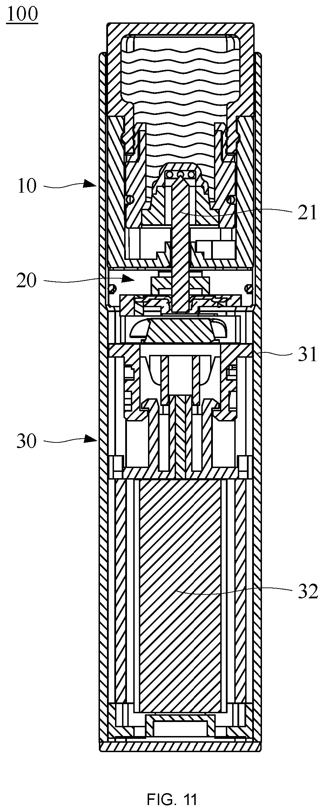

In one embodiment, as shown in FIG. 11, the aromatherapy device 100 further includes a main component 30. The main component 30 is used for controlling the heating state of the heating element 21. The main component 30 includes a housing 31, a power source 32, and a main board (not shown). The liquid storage component 10, the heating component 20, the power source 32, and the main board are mounted on the housing 31. The power source 32 provides electrical energy for the heating element 21. According to presets or user's interaction inputs, the main board can control electrical connection or disconnection between the heating element 21 and the power source 32, so as to control the heating element 21 to start or stop heating. Experiments show that the volatilization rate of the essential oil is positively correlated with its temperature. Therefore, the temperature of the essential oil can be changed by the heating element 21 to adjust the volatilization rate of the essential oil, so that the aromatherapy device 100 is suitable for different usage environments requiring the volatilization rate of essential oils and can meet the requirements of various usage scenarios. For example, the heating power of the heating element 21 can be adjusted through the main board to change the temperature of the essential oil.

Optionally, the heating element 21 is mounted on the main component 30. In one embodiment, as shown in FIG. 11, the liquid storage component 10 is detachably connected to the main component 30, and the heating element 21 is separated from the liquid storage component 10. For example, the connection between the liquid storage component 10 and the main component 30 may be one of adhesion, clamping, or threaded connection. Optionally, the heating element 21 is not connected to the liquid storage component 10, and the two are independently assembled inside the housing 31. When the essential oil in the liquid storage chamber 14 is used up and the liquid storage component 10 needs to be disassembled and replaced, the heating element 21 is not disassembled with the liquid storage component 10, and the heating element 21 can continue to be used without replacement, making the heating element 21 reusable to reduce user's usage cost.

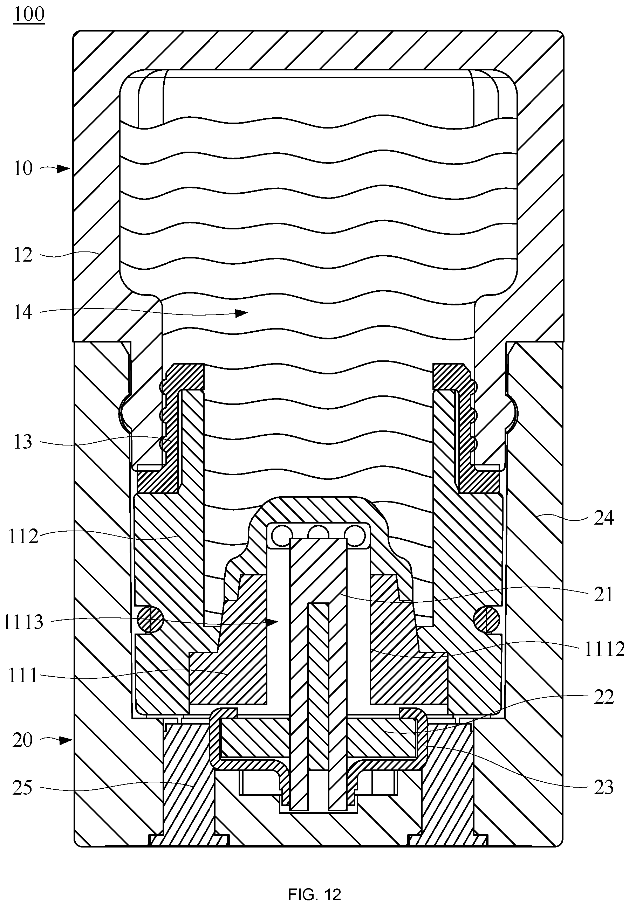

Optionally, the heating element 21 is assembled on the liquid storage component 10. In one embodiment, as shown in FIG. 12, the heating component 20 further includes a mounting bracket 22, a heating element pin 23, a base 24, and an electrode 25. The heating element 21 is arranged on the mounting bracket 22, the heating element pin 23 is electrically connected to the heating element 21, the mounting bracket 22 is mounted on the base 24, and the electrode 25 is inserted into the base 24 and electrically connected to the heating element pin 23. The electrode 25 may be connected to the power source to provide electrical energy for the heating element 21. The base 24 is connected to the liquid storage component 10. For example, the base 24 is connected to the upper shell 12, and the lower shell 11 is accommodated inside the base 24, so that the liquid storage component 10 and the heating component 20 are integrated to facilitate their assembly.

Described above are only some embodiments of the present application, and the scope of protection of the present application is not limited thereto. Any equivalent apparatus or equivalent process transformation made using the description and accompanying drawings of the present application, directly or indirectly applied in other related technical fields, is also included in the scope of patent protection of the present application.

## Claims

1. A liquid storage component used for an aromatherapy device, wherein the liquid storage component is provided with a liquid storage chamber for storing an essential oil; and the liquid storage component comprises a lower shell, the lower shell encloses at least a portion of the liquid storage chamber, the lower shell comprises a liquid guide portion and a support portion, a microporous structure is provided inside the liquid guide portion and is in communication with the liquid storage chamber, the support portion is of a dense structure and is integrally formed with the liquid guide portion, the essential oil enters the microporous structure from the liquid storage chamber and is delivered to a volatilization surface of the liquid guide portion by the microporous structure, and the essential oil can volatilize and diffuse on the volatilization surface into an external environment.

2. The liquid storage component according to claim 1, wherein the liquid guide portion and the support portion are made of ceramic, the liquid guide portion is connected to the support portion, and the liquid guide portion and the support portion are co-fired into a one-piece structure at a preset temperature.

3. The liquid storage component according to claim 2, wherein in the process of fabricating the lower shell, the pre-sintered support portion is placed in a mold, ceramic slurry is injected into the mold to form a lower shell blank comprising the support portion, and the lower shell blank is sintered to obtain the lower shell with a one-piece structure of the ceramic material, wherein a pore forming agent is added to the ceramic slurry.

4. The liquid storage component according to claim 3, wherein the sintering temperature of the support portion is greater than or equal to 1600°C, and the sintering temperature of the liquid guide portion is 500-800°C.

5. The liquid storage component according to claim 2, wherein the liquid guide portion has a pore size of 10-30 µm and a porosity of 30-50%.

6. The liquid storage component according to claim 2, wherein the liquid storage component comprises an upper shell, the upper shell is made of ceramic, the lower shell is connected to one end of the upper shell, the lower shell and the upper shell enclose the liquid storage chamber; and
the support portion is provided with a liquid guide chamber and a liquid guide hole, the liquid guide chamber forms at least a portion of the liquid storage chamber, the liquid guide hole connects the liquid guide chamber with the liquid guide portion, and the essential oil enters the microporous structure via the liquid guide hole.

7. **The** liquid storage component according to claim 6, wherein the support portion is provided with a liquid guide groove; in at least one conventional posture, the liquid guide groove is located at a bottom of the liquid guide chamber, and the liquid guide groove is arranged on a periphery of the liquid guide chamber; the liquid guide hole is formed in a partial area of a wall of the liquid guide groove, and the liquid guide portion seals the liquid guide hole.

8. **The** liquid storage component according to claim 2, wherein the support portion is provided with an air outlet channel, the liquid guide portion is provided with a volatilization space, the volatilization space is in communication with the air outlet channel, and the essential oil in the liquid guide portion volatilizes into the volatilization space and the air outlet channel and diffuses into the external environment.

9. **The** liquid storage component according to claim 8, wherein the lower shell further comprises a partition, the partition is located inside the support portion and connected above the liquid guide portion, and the liquid storage chamber and the air outlet channel are located on two sides of the partition respectively; in the at least one conventional posture, the partition at least partially forms the bottom of the liquid storage chamber, thereby enclosing, together with the support portion, an opening through which the essential oil enters the liquid guide portion.

10. An aromatherapy device, comprising:
the liquid storage component according to any one of claims 1-9 and a heating portion, wherein the heating portion is arranged on the liquid guide portion, and the heating portion is used for heating an essential oil, so that the essential oil is heated at the liquid guide portion to volatilize and diffuse into an external environment.

11. **The** aromatherapy device according to claim 10, wherein the support portion is provided with a mounting chamber, and the liquid guide portion is embedded in the mounting chamber;
the support portion is provided with an air outlet channel, the liquid guide portion is provided with a volatilization space, the volatilization space is in communication with the air outlet channel, and an inner wall, near the volatilization space, of the liquid guide portion forms a volatilization surface; and
the heating portion is embedded on the volatilization surface, a surface of the heating portion is at least partially exposed to the volatilization surface, and the heating portion heats the essential oil in the liquid guide portion, so that the essential oil is heated to volatilize into the volatilization space and the air outlet channel and diffuse into the external environment.

12. **The** aromatherapy device according to claim 11, wherein the area of a cross-section, perpendicular to an extension direction of the heating portion, of the heating portion exposed to the volatilization surface accounts for 30%-70% of the cross-sectional area.

13. **The** aromatherapy device according to claim 10, wherein the resistance value of the heating portion is 0.2-5 Ω.

14. **The** aromatherapy device according to claim 10, wherein the aromatherapy device further comprises a pin portion, and the pin portion is connected with the heating portion into a one-piece structure.
